# EUROPÄISCHE PATENTSCHRIFT

(11) **EP 0 717 614 B1**
(45) Veröffentlichungstag und Bekanntmachung des Hinweises auf die Patenterteilung: **25.11.1998**
(21) Anmeldenummer: 94927594.5
(22) Anmeldetag: 09.09.1994
(51) Int. Cl.: A61K 9/00

(54) **BRAUSEGRANULAT UND VERFAHREN ZU SEINER HERSTELLUNG**
EFFERVESCENT GRANULATED MATERIAL AND METHOD FOR ITS PREPARATION
GRANULAT EFFERVESCENT ET SON PROCEDE DE PREPARATION

(30) Priorität: 09.09.1993 CH 2700/93
(43) Veröffentlichungstag der Anmeldung: 26.06.1996
(73) Patentinhaber: Gergely, Gerhard, Dr., A-1053 Wien (AT)
(72) Erfinder: GERGELY, Gerhard, A-1053 Wien (AT); GERGELY, Thomas, A-1053 Wien (AT); GERGELY, Irmgard, A-1053 Wien (AT); GERGELY, Stefan, A-1050 Wien (AT)
(74) Vertreter: Büchel, Kurt F., Dr.
(86) Internationale Anmeldenummer: EP9403018
(87) Internationale Veröffentlichungsnummer: WO9507070

(56) Entgegenhaltungen:
- EP-A- 0 525 388
- US-A- 4 783 331

## Beschreibung

Die Erfindung betrifft ein Brausegranulat für die Herstellung einer pharmazeutischen Zubereitung mit Zitronensäure und Calciumcarbonat, sowie ein verfahren zu seiner Herstellung. Die Herstellung solcher Produkte ist beispielsweise in der FR-A-2'552'308 und der US-A-4'867'942 beschrieben. Hochdosierte Calciumbrausetabletten, die 2.5 g Calciumcarbonat (entsprechend 1000 mg Calcium) und 4-4.5 g Zitronensäure enthalten, zeigen allerdings, dass Brausetabletten höherer Konzentration von Calciumionen, aufgelöst in 150 oder 200 ml Wasser, dazu neigen, nach längerem Stehen der trinkfertigen Lösung Niederschläge von unlöslichem Tricalciumcitrat zu bilden. Die Zeit bis zur Ausfällung des Tricalciumcitrates und die Menge des Niederschlages ist abhängig von der Konzentration von Calciumionen und Zitronensäure in der trinkfertigen Lösung. Da es aber der Konsument vorzieht, eine Tablette in 150 bis maximal 200 ml Wasser aufzulösen und sehr oft die Lösung nicht sofort getrunken wird, ist diese Erscheinung immer ein Kritikpunkt, umso mehr als auch Brausetabletten, die nur 500 mg Calciumionen in 150 ml Wasser enthalten, eine ebensolche, wenn auch zeitlich wesentlich, d.h. auf bis zu ca. 1 Stunde, verzögerte Fällung zeigen.

Nun wurde in der WO 94/00107 bereits vorgeschlagen, diesen unerwünschten Effekt durch den Ersatz eines bedeutenden Teils (25 bis 73 %) der Zitronensäure durch Äpfelsäure, sowie gegebenenfalls durch verschiedene Salze, zu verzögern. Sie stören - offensichtlich durch teilweise Bindung des Calciumcarbonates und durch Bildung von Mischsalzen - die Bildung von reinem Calciumtricitrat, so dass es wesentlich länger dauert, bis sich das Massenwirkungsgesetz durchsetzt.

Derart grosse Mengen Äpfelsäure verteuern allerdings das Produkt beträchtlich und beschränken die Anwendung im wesentlichen auf Pulvermischungen, die nur durch besondere Massnahmen zu Tabletten verpresst werden können. Die Granulierungseigenschaften der Masse werden nämlich durch diese hohen Zusätze gravierend verschlechtert: die Masse wird sehr teigig. Ausserdem lösen sich solche Tabletten zu langsam, und es kommt bei der Auflösung unter Umständen zu einer Rückstandsbildung, da während des Auflösevorganges die Umsetzung zu z.B. Calciummalat zu langsam erfolgt; dadurch kommt es dann zu Rückständen von nicht reagiertem Calciumcarbonat.

Auch der Zusatz des in der FR-A-2'552'308 (Beispiel 4) vorgeschlagenen Gluconsäure-delta-lactons zeigt den erfindungsgemässen Effekt nur viel zu schwach, da es Gluconsäure erst bei Vorliegen grösserer Mengen an Wasser freisetzt, sodass die für die Herstellung des Brausegranulats (siehe unten) erwarteten Vorteile nicht gegeben sind.

Diese Probleme werden nun in überraschender Weise erstmalig dadurch gelöst, dass 5 bis 20, vorzugsweise 10 bis 15 Gewichtsprozent der Zitronensäure durch wenigstens eine Fremdsäure ersetzt werden, die übrigens ausser Äpfelsäure auch Gluconsäure oder Milchsäure sein kann. Andere Säuren sind deshalb weniger vorteilhaft, weil Weinsäure ein unlösliches Calciumtartrat bildet; Adipinsäure und Ascorbinsäure zeigen den gewünschten Effekt nicht oder nur schwach, und Adipinsäure ist darüber hinaus selbst nur schwer löslich. Andererseits ist auch die angegebene Untergrenze wichtig: auch die beanspruchten Säuren zeigen den gewünschten Effekt nicht oder nur zu schwach, wenn sie in Mengen von nur unter 5 Gewichtsprozent eingesetzt werden.

Bei vielen Versuchen zur Behebung des Problems zeigte es sich, dass bereits ein Ersatz von 100 mg Zitronensäure durch Äpfelsäure bei einer Brausetablette mit 1000 mg Calcium eine Verzögerung der Ausfällzeit in 125 ml Wasser bewirkt: eine sehr stark zur Ausfällung neigende Charge von ursprünglich 7 min wurde um 30% auf 9,5 min, bei einem Zusatz von 200 mg um 50% auf 11 min verbessert. Die Trübung wurde als Absorption photometrisch bei 480 nm auf einen gleichen Wert geprüft, um objektive Parameter zu erhalten.

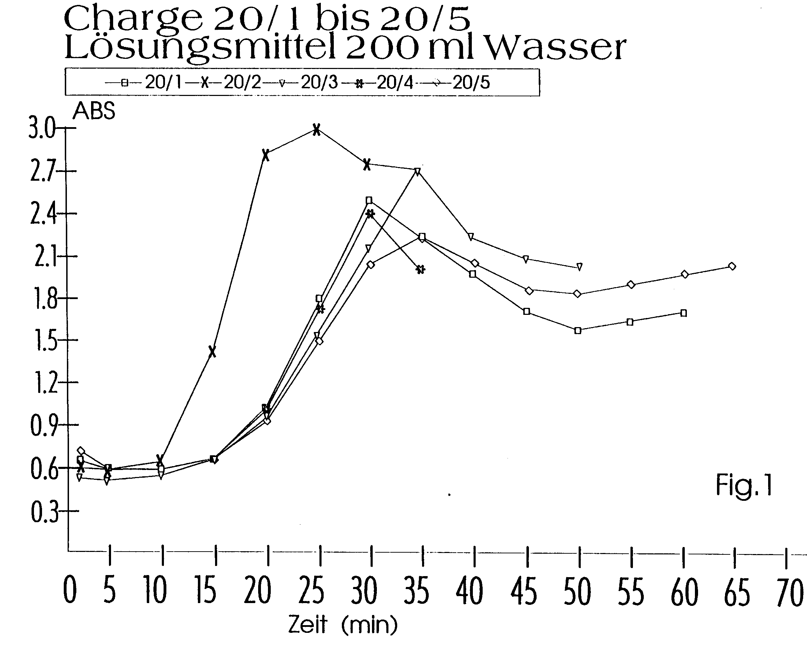

Schon ein Ersatz von nur 5-15 Gew.% der Zitronensäure durch Äpfelsäure verlängert die Ausfällzeit fast auf das Doppelte, zumindest aber um 50% (siehe Fig.1), ohne dass der pH-Wert der Lösung verändert wurde. Es zeigt sich weiters, dass die Fremdsäuren den gleichen Effekt bewirken, unabhängig davon, ob eine Einbindung in das Granulat oder eine trockene Zumischung erfolgt, wenngleich - nicht zuletzt aus verfahrenstechnischer Sicht - eine Granulation zu bevorzugen ist.

Weitere Säuren, die sich als vorteilhaft erwiesen haben, sind Gluconsäure und Milchsäure, welch letztere gleichzeitig als Granulierlösung Verwendung finden kann.

Auch für die Herstellung des Brausegranulates hat sich der Zusatz einer zweiten Säure hinsichtlich der Ausfällung als günstig erwiesen. Bei der Herstellung eines Brausegranulates aus Calcium-carbonat und eventuell Alkalicarbonaten und -bicarbonaten mit Zitronensäure werden grosse Mengen an Lösungen benötigt, um eine Anreaktion von Calciumcarbonat und Zitronensäure zu erhalten; darüber hinaus wirken zusätzlich entweder bei der Granulation oder beim Trocknen erhöhte Temperaturen ein; dadurch kommt es teilweise schon bei der Herstellung des Granulates zur Bildung einer geringen Menge an Tricalciumcitrat, das sich sofort nach dem Auflösen des Granulates oder der Tablette als unlöslicher Rückstand im Glas zeigt.

Daher ist beim Granulieren und Trocknen die Zugabe von Fremdsäuren von Vorteil, weil dadurch die Bildung von Tricalciumcitrat während des Granulierens und Trocknens unterbunden und der Aufbau einer derartigen Brausetablette wesentlich einfacher ohne besondere Vorsichtsmassnahmen möglich wird.

Dabei ist auch die Herstellung eines Granulats in Anwesenheit der Fremdsäure dadurch verbessert, dass es bei Benetzung der Lösungen zum gegenseitigen Auflösen von Zitronensäure und Fremdsäure, also z.B. Äpfelsäure, kommt, wodurch eine Schmelzpunktdepression beider Säuren eintritt, die eine ausserordentlich gute Presshilfe darstellt. Das erlaubt auch einen einfacheren Aufbau des Produktes, so dass man unabhängiger von den Kornstrukturen ist und mit pulverisierter Zitronensäure oder in Mischung mit kristalliner Zitronensäure und pulverisierter Äpfelsäure arbeiten kann, ohne dass durch den verstärkten Kontakt von Calciumcarbonat mit Zitronensäure bei der Granulierungsreaktion bereits Tricalciumcitrat gebildet wird. Durch die erwähnte Schmelzpunkterniedrigung und gegenseitige Durchdringung der Lösungen kann man ausserordentlich elastische und pressfreudige Granulate erzielen.

Die Granulierung kann mit polaren Lösungsmitteln und polaren Lösungsmittelgemischen durchgeführt werden, wobei selbstverständlich auch die Anwendung von Pufferlösungen vorteilhaft ist, um allzu starke Reaktionen zu verhindern.

Weiters kann, wie bereits erwähnt, auch mit einer Lösung von z.B. Äpfelsäure oder Milchsäure in polaren Lösungsmitteln granuliert werden, wobei die Effekte des besseren Granulierverhaltens, des Verhinderns der Bildung von Tricalciumcitrat während der Herstellung, und auch der Verzögerung einer Ausfällung im Glas erhalten bleiben.

Zu diesen Granulaten, oder in diese Granulate eingebaut, können auch die in der Osteoporose-Therapie angewandten Fluorverbindungen zugefügt werden, wie z.B. Natriumfluorid, Natriummonofluorophosphat, Zinkfluorphosphat, etc. Auch Fluor-Apatite können eingebaut werden, wobei die Apatite aufgrund ihrer schweren Löslichkeit in gemahlenem Zustand in der entstehenden Lösung suspendiert und in Schwebe gehalten werden.

### Beispiel 1 (Fig.1, Charge 20/5):

Ersatz von 14% der Gesamtsäure durch Äpfelsäure pulvis und Granulierung mit Wasser/Ethanol, wobei die Zitronensäure ausschliesslich in pulverisierter Form eingesetzt wird:

2500 Gewichtsteile Calciumcarbonat werden mit 3700 Gewichtsteilen pulverisierter Zitronensäure und 600 Gewichtsteilen pulverisierter Äpfelsäure vermischt und bei 45°C mit 200 Gewichtsteilen 70%igem Ethanol 10 min lang unter Reaktion granuliert. Anschliessend wird mittels Vakuum bei einer Temperatur von 60°C getrocknet und zu Tabletten verpresst.

Die Zeit nach dem Auflösen der Tablette bis zum Eintritt einer Trübung durch Tricalciumcitrat betrug 35 min und war damit fast doppelt so lang wie diejenige einer Vergleichstablette ohne Äpfelsäure.

### Beispiel 2 (Charge 20/1 in Fig.1):

Ersatz von 19% der Gesamtsäure durch Äpfelsäure; Einsatz der Zitronensaure in kristalliner und pulverisierter Form, Granulierung mit Wasser:

2500 Gewichtsteile Calciumcarbonat werden mit 2700 Gewichtsteilen kristalliner Zitronensäure, 800 Gewichtsteilen pulverisierter Zitronensäure und 800 Gewichtsteilen Äpfelsäure vermischt, auf 45°C erwärmt, mit 120 Gewichtsteilen Wasser granuliert und abschliessend getrocknet. Das Granulat ist gerade noch gut verpressbar.

Die Ausfällzeit betrug 30 min im Vergleich zu 15 min des Produktes ohne Äpfelsäure; somit konnte eine Verlängerung von 100% erreicht werden.

### Beispiel 3 (Charge 20/4 der Fig.1):

Ersatz von 9% der Gesamtsäure durch Äpfelsäure: ähnlich wie in Beispiel 2 können auch nur 400 Gewichtsteile der Gesamtsäure durch Äpfelsäure ersetzt werden; die Mischung wird dann in zwei Stufen mit einer Pufferlösung (z.B. einer Vorreaktionslösung entsprechend dem US Patent Nr. 4,867,942) granuliert, die wie folgt hergestellt wird: 130 Gewichtsteile Zitronensäure und 27 Gewichtsteile Calciumcarbonat werden in 180 Gewichtsteilen Wasser gelöst. Das Produkt 20/4 zeigt in Fig.1 bei der Absorption von 2,1 im Vergleich zum Produkt ohne Äpfelsäure (Charge 20/2) eine Verlängerung der Ausfällzeit von 18 auf fast 30 min.

### Beispiel 4 (Charge 20/3 in Fig.1):

Es kann wie bei Beispiel 3 vorgegangen werden, wobei die ganzen 400 Gewichtsteile der Äpfelsäure in der als Pufferlösung wirkenden Granulierflüssigkeit von 300 Gewichtsteilen einer Ethanol-Wasser-Mischung (1:1) gelöst sind. Das Endprodukt zeigt hinsichtlich der Zeit bis zur beginnenden Bildung des Tricalciumcitrats gleiches Verhalten wie dasjenige des Beispiels 3.

### Beispiel 5:

Es wird wie in Beispiel 4 gearbeitet, mit dem Unterschied, dass die Pufferlösung anstelle der Äpfelsäure aus 250 Gewichtsteilen Glukonsäure oder Milchsäure (auf Trockensubstanz gerechnet) durch Verdünnung mit 100 ml Wasser hergestellt wird. Das Endprodukt zeigt hinsichtlich der Zeit bis zur beginnenden Bildung des Tricalciumcitrats gleiches Verhalten wie dasjenige des Beispiels 4.

## Patentansprüche

1. Brausegranulat für die Herstellung einer pharmazeutischen Zubereitung, enthaltend Calciumcarbonat und Zitronensäure, sowie eine weitere, feste, essbare, organische Säure und/oder deren Salze, dadurch gekennzeichnet, dass 5 - 20, vorzugsweise 10 bis 15 Gewichtsprozent der für die Reaktion mit dem Calciumcarbonat vorgesehenen Menge der Gesamtmenge der organischen Säuren bzw. Salze durch wenigstens eine der folgenden Verbindungen ersetzt sind: Äpfelsäure, Gluconsäure und Milchsäure, sowie deren saure Salze.

2. Brausegranulat nach Anspruch 1, dadurch gekennzeichnet, dass die organischen Säuren von wenigstens einem Teil des Calciumcarbonates abgedeckt sind, sowie mit ihm teilreagiert und granuliert vorliegen.

3. Brausegranulat nach Anspruch 1 oder 2, dadurch gekennzeichnet, dass es ausserdem pharmazeutisch wirksame und zulässige Mengen wenigstens einer Fluor-Verbindung enthält.

4. Verfahren zur Herstellung eines Brausegranulates nach Anspruch 1 oder 2, bei dem Zitronensäure und Calciumcarbonat unter teilweiser Anreaktion granuliert werden, dadurch gekennzeichnet, dass wenigstens ein Teil der die Zitronensäure teilweise ersetzenden Verbindung in fester, pulverisierter Form mit der Zitronensäure und dem Calciumcarbonat gemischt und unter Befeuchtung gemeinsam granuliert wird.

5. Verfahren zur Herstellung eines Brausegranulates nach Anspruch 1, bei dem Zitronensäure und Calciumcarbonat unter teilweiser Anreaktion granuliert werden, dadurch gekennzeichnet, dass wenigstens ein Teil der die Zitronensäure teilweise ersetzenden Verbindung in der Granulierungsflüssigkeit, vorzugsweise in einer Pufferlösung, gelöst ist.

## Claims

1. Effervescent granulated material for the preparation of a pharmaceutical formulation, containing calcium carbonate and citric acid as well as a further, solid, edible, organic acid and/or salts thereof, characterized in that 5 - 20, preferably 10 to 15, percent by weight of the total amount of the organic acids or salts intended for the reaction of the calcium carbonate are replaced by at least one of the following compounds: malic acid, gluconic acid and lactic acid and acidic salts thereof.

2. Effervescent granulated material according to Claim 1, characterized in that the organic acids are covered by at least a part of the calcium carbonate and undergo partial reaction with it and are present in granulated form.

3. Effervescent granulated material according to Claim 1 or 2, characterized in that it furthermore contains a pharmaceutically effective and permissible amount of at least one fluorine compound.

4. Process for the preparation of an effervescent granulated material according to Claim 1 or 2, in which citric acid and calcium carbonate are granulated with partial reaction, characterized in that at least a part of the compound partially replacing the citric acid is mixed in solid, powdered form with the citric acid and the calcium carbonate and granulated together with moistening.

5. Process for the preparation of an effervescent granulated material according to Claim 1, in which citric acid and calcium carbonate are granulated with partial reaction, characterized in that at least a part of the compound partially replacing the citric acid is dissolved in the granulating liquid, preferably in a buffer solution.

## Revendications

1. Granulat effervescent pour la fabrication d'une préparation pharmaceutique, contenant du carbonate de calcium et de l'acide citrique, ainsi qu'un autre acide organique, comestible, solide, et/ou ses sels, caractérisé en ce que 5-20, de préférence 10 à 15 % en poids de la quantité, prévue pour la réaction avec le carbonate de calcium, de la quantité totale des acides organiques, respectivement des sels organiques, sont remplacés par au moins l'une des combinaisons ci-après : acide malique, acide gluconique et acide lactique, ainsi que leurs sels acides.

2. Granulat effervescent selon la revendication 1, caractérisé en ce que les acides organiques sont recouverts par au moins une partie du carbonate de calcium, et se présentent sous une forme ayant partiellement réagi avec lui et granulée.

3. Granulat effervescent selon la revendication 1 ou 2, caractérisé en ce qu'il contient, en outre, des quantités, pharmaceutiquement efficaces et admissibles, d'au moins une combinaison fluor.

4. Procédé de préparation d'un granulat effervescent selon la revendication 1 ou 2, dans lequel on granule l'acide citrique et le carbonate de calcium avec une mise en réaction partielle, caractérisé en ce qu'au moins une partie de la combinaison remplaçant partiellement l'acide citrique est mélangée, sous une forme solide pulvérisée, à l'acide citrique et au carbonate de calcium, et est granulée conjointement, avec humidification.

5. Procédé de préparation d'un granulat effervescent selon la revendication 1, dans lequel l'acide citrique et le carbonate de calcium sont granulés avec une mise en réaction partielle, caractérisé en ce qu'au moins une partie de la combinaison remplaçant partiellement l'acide citrique est dissoute dans le liquide de granulation, de préférence en une solution tampon.
